# EUROPÄISCHE PATENTSCHRIFT

(11) **EP 2 151 254 B1**
(45) Veröffentlichungstag und Bekanntmachung des Hinweises auf die Patenterteilung: **09.08.2017**
(21) Anmeldenummer: 09163908.8
(22) Anmeldetag: 26.06.2009
(51) Int. Cl.: A61L 31/02, A61L 31/10, A61L 31/14, A61L 31/16

(54) **Biokorrodierbares Implantat mit einer Beschichtung enthaltend eine wirkstoff-tragende Polymermatrix**
Bio-corrodable implant with a coating containing a polymer matrix carrying an active agent
Implant biocorrodable doté d'un revêtement contenant une matrice polymère portant une matière active

(30) Priorität: 28.07.2008 DE 102008040786
(43) Veröffentlichungstag der Anmeldung: 10.02.2010
(73) Patentinhaber: Biotronik VI Patent AG, 6341 Baar (CH)
(72) Erfinder: Borck, Alexander, 91086, Aurachtal (DE); Adden, Nina, 90427, Nürnberg (DE)
(74) Vertreter: Randoll, Sören

(56) Entgegenhaltungen:
- WO-A1-02/05864
- US-A1- 2006 165 751
- US-A1- 2008 058 923

## Beschreibung

### Technisches Gebiet

Die Erfindung betrifft ein biokorrodierbares Implantat mit einer Beschichtung enthaltend eine Wirkstoff-tragende Polymermatrix.

### Technologischer Hintergrund und Stand der Technik

Implantate haben in vielfältiger Ausführungsform Anwendung in der modernen Medizintechnik gefunden. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z.B. als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z.B. durch Röntgenuntersuchungen erfolgen.

Das Implantat oder der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Ein aussichtsreicher Ansatz zur Lösung des Problems liegt in der Verwendung biokorrodierbarer Metalle und deren Legierungen als Implantatwerkstoff, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen. Solche biokorrodierbaren Implantate und Stents weisen häufig auch eine Beschichtung oder Kavitätenfüllung mit einem geeigneten Polymer auf.

Ein Problem beim Einsatz dieser biokorrodierbaren Implantate, die ganz oder in Teilen aus einem metallischen Werkstoff bestehen, liegt darin, dass die Abbauprodukte, die bei der Korrosion des Implantats entstehen und freigesetzt werden, oft einen merklichen Einfluss auf den lokalen pH-Wert haben und sowohl zu ungewünschten Gewebsreaktionen führen können, als auch einen unerwünschten Einfluss auf die weitere Korrosionsrate des Implantats ausüben können. Insbesondere beim Abbau von Mg-haltigen biokorrodierbaren Implantatwerkstoffen kann es zu einem Anstieg des pH-Wertes in der unmittelbaren Umgebung kommen. Dieser pH-Wertanstieg kann zu einem Phänomen führen, dass unter dem Begriff Alkalose zusammengefasst wird. Der lokale pH-Wertanstieg führt dabei zu einem Ungleichgewicht der Ladungsverteilung in den das Gefäß umgebenden Muskelzellen, was dazu führen kann, dass es zu einer lokalen Erhöhung des Muskeltonus im Bereich des Implantats kommt. Dieser erhöhte Druck auf das Implantat kann zum vorzeitigen Verlust der Implantatintegrität führen.

Dokument EP 1 795 215 A2 offenbart ein Implantat (Stent) mit einem Grundkörper, der ganz oder in Teilen aus einem biokorrodierbaren, metallischen Werkstoff besteht (Magnesium), wobei der Werkstoff so beschaffen ist, dass er in wässriger Umgebung zu einem alkalischen Produkt zersetzt wird und wobei der Grundkörper eine Beschichtung aufweist, die aus einem säureabgebenden Polymer besteht oder dieses enthält.

Aufgabe der vorliegenden Erfindung war es einen oder mehrere der geschilderten Nachteile des Standes der Technik zu mindern oder zu überwinden.

### Erfindungsgemäße Lösung

Die Aufgabe wird durch Bereitstellung eines Implantats gelöst, mit einem Grundkörper, der ganz oder in Teilen aus einem biokorrodierbaren, metallischen Werkstoff besteht, wobei der Werkstoff so beschaffen ist, dass er in wässriger Umgebung zu einem alkalischen Produkt zersetzt wird und wobei der Grundkörper eine Beschichtung oder eine Kavitätenfüllung aufweist, die eine Polymermatrix und mindestens einen in die Polymermatrix eingebetteten Wirkstoff umfasst, dadurch gekennzeichnet, dass mindestens ein Polymer der Matrix und der mindestens eine Wirkstoff so aufeinander abgestimmt sind, dass bei erhöhtem pH-Wert die Freisetzungsrate des Wirkstoffs aus der Matrix erhöht ist. Ein Vorteil der erfindungsgemäßen Lösung liegt darin, dass die eingebetteten Wirkstoffe sowohl zeitlich verzögert als auch räumlich begrenzt, erst bei Vorliegen eines lokal erhöhten pH-Wertes, vermehrt aus der Polymermatrix freigesetzt werden.

Bei Einsatz des erfindungsgemäßen Implantats ist es nicht mehr notwendig z.B. einer möglichen Alkalose durch systemische Verabreichung von Medikamenten oder Wirkstoffen entgegenzuwirken. Entsprechende Wirkstoffe können bereits in die Beschichtung des biokorrodierbaren Implantats eingebettet sein und werden bei Änderung des lokalen pH-Wertes vor Ort vermehrt freigesetzt. Somit können insgesamt deutlich geringere Wirkstoffmengen eingesetzt werden, die dann bevorzugt am gewünschten Ort und zum Zeitpunkt des Bedarfs zur Verfügung gestellt werden. Der Patient wird weniger belastet und die Behandlungskosten sind reduziert.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Der Stent kann vor oder nach dem Crimpen auf einen Ballon beschichtet werden.

Gemäß einer ersten Variante weist der Grundkörper des Implantats demnach eine Beschichtung auf, die eine erfindungsgemäße Polymermatrix und mindestens einen in die Polymermatrix eingebetteten Wirkstoff enthält oder aus dieser besteht. Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 30 µm. Der Gewichtsanteil der erfindungsgemäßen Polymermatrix an den die Beschichtung bildenden Komponenten der Beschichtung beträgt vorzugsweise mindestens 40%, besonders bevorzugt mindestens 70%. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel sogenannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Alternativ kann die Polymermatrix, umfassend den mindestens einen in die Polymermatrix eingebetteten Wirkstoff, als Kavitätenfüllung vorliegen oder Bestandteil einer Kavitätenfüllung sein. Das Implantat, insbesondere der Stent, weist zu diesem Zweck eine oder mehrere Kavitäten auf. Kavitäten befinden sich beispielsweise an der Oberfläche des Implantats und lassen sich durch Laserablation in Mikrometerdimensionen erstellen. Bei Implantaten, insbesondere Stents, mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Biokorrodierbare metallische Werkstoffe im Sinne der Erfindung umfassen Metalle und Legierungen ausgewählt aus der Gruppe umfassend Eisen, Wolfram, Zink, Molybdän und Magnesium und insbesondere solche biokorrodierbare metallische Werkstoffe, die in wässriger Lösung zu einem alkalischen Produkt korrodieren.

Vorzugsweise besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Insbesondere ist der biokorrodierbare metallische Werkstoff eine Magnesiumlegierung.

Unter einer biokorrodierbare Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist so in ihrer Zusammensetzung zu wählen, dass sie biokorrodierbar ist. Als Prüfmedium zur Testung des Korrosionsverhaltens von Legierungen dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Erfindungsgemäß sind das mindestens eine Polymer der Polymermatrix und der mindestens eine Wirkstoff so aufeinander abgestimmt, dass bei erhöhtem pH-Wert die Freisetzungsrate des Wirkstoffs aus der Polymermatrix erhöht ist.

Ein solcher erhöhter pH-Wert liegt dann vor, wenn der lokale pH-Wert, gegenüber dem physiologischen pH-Wert in den basischen pH-Wertbereich verändert ist. Insbesondere liegt dann ein erhöhter pH-Wert im Sinne der Erfindung vor, wenn der pH-Wert in der lokalen Umgebung größer als 8 ist.

Unter dem Begriff "Freisetzungsrate" wird im Sinne der Erfindung die aus der Polymermatrix freigesetzte Menge an Wirkstoff pro Zeiteinheit verstanden. Geeignete Methoden und Messverfahren zur Bestimmung der Freisetzungsrate sind dem Fachmann bekannt, insbesondere eignen sich solche Messverfahren, wie sie sich im Bereich der Galenik bereits vielfach bewährt haben.

In einer bevorzugten Ausführungsform ist die Freisetzungsrate des Wirkstoffs aus der Polymermatrix bei einem pH-Wert größer 8 erhöht. In besonders bevorzugten Ausführungsformen weist der Wirkstoff bei einem pH-Wert größer 8 eine mindestens 2-fach erhöhte Freisetzungsrate aus der Polymermatrix auf im Vergleich zur Freisetzungsrate bei physiologischem pH-Wert.

Das mindestens eine Polymer der Polymermatrix kann pH-Wert abhängige Eigenschaften besitzen. Das Polymer weist beispielsweise mindestens eine funktionelle Gruppe auf, die mit steigendem pH-Wert einen Übergang zwischen einem neutralen und einem ionischen Ladungszustand zeigt. Dabei kann die mindestens eine funktionelle Gruppe des Polymer bei physiologischem pH-Wert in ionischem Ladungszustand und bei, im Sinne der Erfindung, erhöhtem pH-Wert in neutralem Ladungszustand vorliegen. Es ist auch möglich, dass die mindestens eine funktionelle Gruppe bei physiologischem pH-Wert in neutralem Zustand und bei erhöhtem pH-Wert in einem ionischen Ladungszustand vorliegt. In einem solchen System kann der Wirkstoff durch Veränderung der Ladungszustände in der Polymermatrix beim Übergang von einem physiologischen pH-Wert zu einem erhöhten pH-Wert vermehrt freigesetzt werden. Das mindestens eine Polymer kann mehrere gleiche oder verschiedene funktionelle Gruppen aufweisen, die bei physiologischem pH-Wert nicht alle im selben Ladungszustand vorliegen müssen.

In einer bevorzugten Ausführungsform wird die mindestens eine funktionelle Gruppe ausgewählt aus der Gruppe umfassend eine Carbonsäurefunktion, eine Aminfunktion oder eine Amidfunktion.

In einer Ausführungsform der Erfindung umfasst die Polymermatrix ein Hydrogel. Ein Hydrogel ist ein wasserenthaltendes, aber wasserunlösliches Polymer, dessen Moleküle chemisch, z.B. durch kovalente oder ionische Bindungen oder physikalisch, z.B. durch Verschlaufen der Polymerketten, zu einem dreidimensionalen Netzwerk verknüpft sind. Erfindungsgemäße Hydrogele sind in der Lage bei Änderung des pH-Wertes ihr Volumen zu verändern, in dem sie bei erhöhtem pH-Wert entweder unter Volumenzunahme vermehrt Wasser aufnehmen oder unter Volumenabnahme weniger Wasser aufnehmen können. Diese Hydrogele können beispielsweise hergestellt werden durch Umsetzung ethylenisch ungesättigter Monomere und Polymere, die ionisierbare Gruppen tragen, mit Vernetzern und Polymerisationskatalysatoren. Alternativ dazu können geeignete Hydrogele hergestellt werden durch Kondensationsreaktionen mit difunktionellen und mehrfunktionellen Monomeren. Dem Fachmann sind geeignete Monomere und Polymere sowie Verfahren zu deren Herstellung bekannt. Ebenso sind dem Fachmann Methoden und Verfahren zur Herstellung von geeigneten Hydrogelen mittels solcher Mono- oder/und Polymere bekannt. Hydrogele expandieren durch Volumenzunahme bei erhöhtem pH-Wert z.B. wenn das Hydrogel Carboxylgruppen enthält. Eine Volumenabnahme des Hydrogels bei erhöhtem pH-Wert kann z.B. dann erfolgen, wenn das Netzwerk Amingruppen und/oder Amidgruppen enthält.

Bevorzugte Hydrogele enthalten ein Polymer auf Basis von Acrylsäure, Methacrylsäure oder einem Derivat von Acrylsäure oder Methacrylsäure.

Erfindungsgemäß kann der Wirkstoff beispielsweise als Prodrug in die Polymermatrix eingebettet sein. Der Wirkstoff liegt zunächst gekoppelt an Makromoleküle vor, die den Wirkstoff in der Polymermatrix eingebettet halten. Solche Makromoleküle sind dem Fachmann bekannt, insbesondere kann das Makromolekül Dextran sein. Auch das Mono- oder Polymer der Polymermatrix kann ein solches Makromolekül im Sinne der Erfindung sein. Das Prodrugsystem zeichnet sich dadurch aus, dass der über chemische Bindungen an das Makromolekül gekoppelte Wirkstoff durch das Vorliegen eines erhöhten pH-Wertes aus der Polymermatrix freigesetzt wird. Dabei werden die chemischen Bindungen, die den Wirkstoff mit dem Makromolekül verbinden, gelöst und der Wirkstoff kann aus der Polymermatrix austreten. Bevorzugt ist der Wirkstoff über chemische Bindungen in der Polymermatrix fixiert, die basenkatalytisch gespalten werden können, besonders bevorzugt über Esterbindungen, wie z.B. Sulfonsäureester, oder Amidbindungen. Im Folgenden ist ein Beispiel für ein geeignetes Prodrugsystem gegeben: Makromolekül-PhSO₂Cl + Bosentan-OH → Makromolekül-PhSO₂O-Bosentan

Die Rückreaktion unter Freisetzung des Wirkstoffs Bosentan findet dann bei Anwesenheit von Hydroxidionen bei erhöhtem pH-Wert statt.

Ein Prodrugsystem im Sinne der Erfindung liegt auch vor, wenn der Wirkstoff zunächst verkapselt vorliegt und diese wirkstofftragenden Kapseln in der Polymermatrix eingebettet vorliegen. Der Wirkstoff wird dann bei erhöhtem pH-Wert aus der Kapsel, aus der Polymermatrix vermehrt freigesetzt. Dem Fachmann sind geeignete Formulierungen solcher Verkapselungen insbesondere aus dem Bereich der Galenik bekannt.

Als Wirkstoff kann jeder bekannte Wirkstoff eingesetzt werden, der so mit der Polymermatrix der Beschichtung oder Kavitätenfüllung des Implantats interagiert, dass bei erhöhtem pH-Wert die Freisetzungsrate des Wirkstoffs erhöht ist. Bevorzugt kommen solche Wirkstoffe zum Einsatz, die geeignet sind zur Behandlung oder Prophylaxe einer Alkalose. Solche Wirkstoffe sind ausgewählt aus der Gruppe enthaltend Vasodilatoren, Entzündungshemmer und Wirkstoffe zur lokalen pH-Wertregulation. Besonders bevorzugte Wirkstoffe sind ausgewählt aus der Gruppe enthaltend NO-freisetzende Substanzen und Bosentan®, Dipyridamol, dODN oder allgemein Endothelin-Rezeptor-Antagonisten, Kalziumkanalblocker wie Amlodipin, Nifidipin oder Verapamil.

Das erfindungsgemäße Implantat kann eine weitere, äußere Beschichtung aufweisen. Eine solche weitere äußere Schicht kann die Beschichtung oder Kavitätenfüllung umfassend eine Polymermatrix und mindestens einen Wirkstoff ganz oder in Teilen bedecken. Diese äußere Beschichtung kann ein degradierendes Polymer enthalten oder daraus bestehen, insbesondere ein Polymer aus der Klasse der PLGA (poly(lactic-co-glycolic acid)) oder der PLGA-PEG Blockcopolymere. Ggf. kann in diese weitere, äußere Schicht ein Wirkstoff eingebettet sein, der frei eluieren kann oder bei Degradation der äußeren Beschichtung freigesetzt wird. Eine solche weitere, äußere Beschichtung kann dazu verwendet werden in einem Mehrschichtensystem die durch Änderung des pH-Wertes vermittelte Freisetzung des mindestens einen Wirkstoffs aus der Polymermatrix zu verzögern. Zunächst wird die weitere äußere Beschichtung degradiert, erst dann ist die innere Beschichtung zugänglich, die dann bei Vorliegen eines erhöhten pH-Wertes den mindestens einen Wirkstoff freisetzt.

Nachfolgend wird die Erfindung anhand von Ausführungsbeispielen näher erläutert.

### Ausführungsbeispiel 1 - Polyacrylsäure mit Bosentan

5,0g (69mmol) Acrylsäure werden in 100ml Wasser bei Raumtemperatur gelöst und unter Rühren für 30min mit N₂ entgast. Die Polymerisation wird gestartet durch Zugabe von 1mol% 2,2'-Azobis(2-amidinopropan)dihydrochlorid und Erhitzen auf 60°C. Die Polymerisation wird für 12h durchgeführt. Nach dem Abkühlen auf Raumtemperatur wird die viskose Lösung gegen Wasser dialysiert (molecular cut off (MCO) 13.000 Da). Die erhaltene Polyacrylsäure zeigt in wässriger Umgebung ein mit steigendem pH-Wert erhöhtes Quellvermögen.

### Matrixpräparation und Wirkstoffeinbau:

1 g des so erhaltenen Polymers werden mit 30% Bosentan versetzt.

### Ausführungsbeispiel 2 - Poly(N-isopropylacrylamid-co-allylamine) mit Verapamil

3,8g (33,6mmol) N-isopropylacrylamid (NIPAM) und 0,2g (3,4mmol) Allylamin (10% des NIPAM Monomers) werden in 230 ml THF (Tetrahydrofuran) bei Raumtemperatur gelöst. Es folgt die Zugabe von 0,06% SDS und 0,067g (1,3mol%; 0,44mmol) N,N'-methylen-bis-acrylamid. Die Lösung wird unter Rühren für 30min mit N2 entgast und auf 70°C erwärmt. 0,166g Kaliumpersulfat werden in 20ml Wasser gelöst und zur Reaktion gegeben um die Reaktion zu starten. Die Reaktion wird bei 68-70°C für 4h durchgeführt. Nach Abkühlen auf Raumtemperatur wird der Niederschlag für 5 Tage gegen Wasser dialysiert (molecular cut off (MCO) 13.000 Da). Das erhaltene Poly(N-isopropylacrylamid-co-allylamin) zeigt in wässriger Umgebung ein mit steigendem pH-Wert verringertes Quellvermögen.

### Matrixpräparation und Wirkstoffeinbau:

1g des so erhaltenen Polymers werden mit Verapamil versetzt und mit 0,04g (25wt%) Glutaraldehyd für 2h bei Raumtemperatur vernetzt.

Alternativ kann die Matrixpräparation und Wirkstoffeinbettung wie folgt ausgeführt werden: 1g des so erhaltenen Polymers werden mit etwa 300 mg Verapamil versetzt. Anschließend werden 0,032g (0,17mmol) 1-Ethyl-3-(3-dimethylaminopropyl)carbodiimid gelöst in 200µl Wasser und 0,015g (0.085mmol) Adipinsäuredihydrazid ebenfalls gelöst in 200µl Wasser bei zugesetzt und für 2h gerührt.

### Ausführungsbeispiel 3 - Beschichtung eines Stents

Ein Stent aus der biokorrodierbaren Magnesiumlegierung WE43 (4 Gew.% Yttrium, 3 Gew.% Seltenerdmetalle außer Yttrium, Rest Magnesium und herstellungsbedingte Verunreinigungen) wird wie folgt beschichtet:
Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit einer der Polymermischungen aus Ausführungsbeispiel 1 oder 2 beschichtet. Bei einem Düsenabstand von 20 mm ist ein 18 mm langer Stent nach ca. 10 min beschichtet. Nach Erreichen der beabsichtigten Schichtmasse wird der Stent 5 min bei Raumtemperatur getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet. Eine Schichtdicke der aufgetragenen Beschichtung beträgt etwa 10 µm.

### Ausführungsbeispiel 4 - Mehrschichtensystem

Ein Stent aus der biokorrodierbaren Magnesiumlegierung WE43 (4 Gew.% Yttrium, 3 Gew.% Seltenerdmetalle außer Yttrium, Rest Magnesium und herstellungsbedingte Verunreinigungen) wird erst mit einer Lösung von hochmolekularem PLLA (Poly-L-lactid) (Boehringer Ingelheim; M_{w} 300.000) und Bosentan® (9:1) in Chloroform beschichtet. Dazu wird der Stent von Staub und Rückständen gereinigt und in eine geeignete Stentbeschichtungsapparatur (DES Coater, Eigenentwicklung Firma Biotronik) eingespannt. Nach Erreichen der beabsichtigten Schichtmasse von ca. 400µg wird der Stent bei Raumtemperatur im Vakuum getrocknet und es wird eine zweite Polymerschicht aus einem PLGA-PEG-Blockcopolymer (Boehringer Ingelheim) aufgesprüht.

## Patentansprüche

1. Implantat mit einem Grundkörper, der ganz oder in Teilen aus einem biokorrodierbaren, metallischen Werkstoff besteht, wobei der Werkstoff so beschaffen ist, dass er in wässriger Umgebung zu einem alkalischen Produkt zersetzt wird und wobei der Grundkörper eine Beschichtung oder eine Kavitätenfüllung aufweist, die eine Polymermatrix und mindestens einen in die Polymermatrix eingebetteten Wirkstoff umfasst, **dadurch gekennzeichnet, dass** mindestens ein Polymer der Matrix und der mindestens eine Wirkstoff so aufeinander abgestimmt sind, dass bei erhöhtem pH-Wert die Freisetzungsrate des Wirkstoffs aus der Matrix erhöht ist.

2. Implantat nach Anspruch 1, wobei das Implantat ein Stent ist.

3. Implantat nach einem der Ansprüche 1 und 2, bei dem der biokorrodierbare, metallische Werkstoff eine Magnesiumlegierung ist.

4. Implantat nach einem der vorhergehenden Ansprüche, wobei die Freisetzungsrate des Wirkstoffs aus der Polymermatrix bei einem pH-Wert größer als 8 erhöht ist.

5. Implantat nach einem der vorhergehenden Ansprüche, wobei der Wirkstoff bei einem pH-Wert größer als 8 eine mindestens 2-fach erhöhte Freisetzungsrate aus der Polymermatrix aufweist.

6. Implantat nach einem der vorhergehenden Ansprüche, wobei das Polymer mindestens eine funktionelle Gruppe aufweist, die mit steigendem pH-Wert einen Übergang zwischen einem ionischen und einem neutralen Ladungszustand zeigt.

7. Implantat nach Anspruch 6, bei dem die funktionelle Gruppe ausgewählt ist aus der Gruppe umfassend eine Carbonsäurefunktion, eine Aminfunktion und eine Amidfunktion.

8. Implantat nach einem der vorhergehenden Ansprüche, bei dem die Polymermatrix ein Hydrogel umfasst.

9. Implantat nach Anspruch 8, bei dem das Hydrogel ein Polymer auf Basis von Acrylsäure, Methacrylsäure oder einem Derivat von Acrylsäure oder Methacrylsäure ist.

10. Implantat nach einem der vorhergehenden Ansprüche, wobei das Implantat eine weitere, äußere Beschichtung aufweist, die ein degradierendes Polymer enthält.

11. Implantat nach einem der vorhergehenden Ansprüche, bei dem der Wirkstoff als Prodrug in die Polymermatrix eingebettet ist.

12. Implantat nach Anspruch 11, bei dem der Wirkstoff über chemische Bindungen in der Polymermatrix fixiert vorliegt, die basenkatalytisch gespalten werden.

13. Implantat nach einem der vorhergehenden Ansprüche, bei dem der Wirkstoff ausgewählt ist aus der Gruppe enthalten Vasodilatoren, Entzündungshemmer und Wirkstoffe zur pH-Wertregulation.

14. Implantat nach einem der vorhergehenden Ansprüche, wobei der Wirkstoff ausgewählt ist aus der Gruppe enthaltend NO-freisetzende Substanzen und Bosentan®, Dipyridamol, dODN oder allgemein Endothelin-Rezeptor-Antagonisten, Kalziumkanalblocker wie Amlodipin, Nifidipin oder Verapamil.

## Claims

1. An implant with a main body consisting completely or partially of a biocorrodible metallic material, wherein the material is such that it decomposes in an aqueous environment to form an alkaline product, and wherein the main body has a coating or a cavity filling comprising a polymer matrix and at least one active substance embedded in the polymer matrix, **characterised in that** at least one polymer of the matrix and the at least one active substance are coordinated so that the active substance release rate from the matrix is increased at an elevated pH.

2. The implant according to claim 1, wherein the implant is a stent.

3. The implant according to any one of claims 1 and 2, wherein the biocorrodible metallic material is a magnesium alloy.

4. The implant according to any one of the preceding claims, wherein the active substance release rate from the polymer matrix is increased at a pH above 8.

5. The implant according to any one of the preceding claims, wherein the active substance release rate from the polymer matrix is increased by a factor of at least 2 when the pH is higher than 8.

6. The implant according to any one of the preceding claims, wherein the polymer has at least one functional group which shows a transition between an ionic charge state and a neutral charge state when there is an increase in pH.

7. The implant according to claim 6, wherein the functional group is selected from the group comprising a carboxylic acid function, an amine function and an amide function.

8. The implant according to any one of the preceding claims, wherein the polymer matrix comprises a hydrogel.

9. The implant according to claim 8, wherein the hydrogel is selected from a polymer based on acrylic acid, methacrylic acid, or a derivative of acrylic acid or methacrylic acid.

10. The implant according to any one of the preceding claims, wherein the implant has an additional outer coating containing a degradable polymer.

11. The implant according to any one of the preceding claims, wherein the active substance is embedded as a prodrug in the polymer matrix.

12. The implant according to claim 11, wherein the active substance is fixed in the polymer matrix by chemical bonds cleaved by base catalysis.

13. The implant according to any one of the preceding claims, wherein the active substance is selected from the group comprising vasodilators, anti-inflammatories and pH-regulating active substances.

14. The implant according to any one of the preceding claims, wherein the active substance is selected from the group comprising NO-releasing substances and Bosentan®, dipyridamol, dODN, or generally endothelin receptor antagonists, or calcium channel blockers such as amlodipine, nifidipine and verapamil.

## Revendications

1. Implant avec un corps de base qui est constitué totalement ou en partie d'un matériau métallique biocorrodable, où le matériau est tel qu'il est décomposé en un produit alcalin dans un environnement aqueux et où le corps de base présente un revêtement ou un remplissage de cavités qui comprend une matrice polymère et au moins une matière active incorporée dans la matrice polymère, **caractérisé en ce qu'**au moins un polymère de la matrice et l'au moins une matière active sont compatibles l'un par rapport à l'autre de sorte que, pour une valeur élevée de pH, le taux de libération de la matière active à partir de la matrice est augmenté.

2. Implant selon la revendication 1, où l'implant est un stent.

3. Implant selon l'une des revendications 1 et 2, chez lequel le matériau métallique biocorrodable est un alliage de magnésium.

4. Implant selon l'une des revendications précédentes, dans lequel le taux de libération de la matière active à partir de la matrice polymère est augmenté pour une valeur de pH supérieure à 8.

5. Implant selon l'une des revendications précédentes, dans lequel la matière active présente un taux de libération à partir de la matrice polymère augmenté d'au moins 2 fois pour une valeur de pH supérieure à 8.

6. Implant selon l'une des revendications précédentes, dans lequel le polymère présente au moins un groupe fonctionnel qui présente une transition entre un état de charge ionique et un état de charge neutre avec une valeur de pH en progression.

7. Implant selon la revendication 6, chez lequel le groupe fonctionnel est choisi dans le groupe comprenant une fonction acide carboxylique, une fonction amine et une fonction amide.

8. Implant selon l'une des revendications précédentes, chez lequel la matrice polymère comprend un hydrogel.

9. Implant selon la revendication 8, chez lequel l'hydrogel est un polymère à base d'acide acrylique, d'acide méthacrylique ou d'un dérivé d'acide acrylique ou d'acide méthacrylique.

10. Implant selon l'une des revendications précédentes, où l'implant présente un nouveau revêtement externe qui contient un polymère de dégradation.

11. Implant selon l'une des revendications précédentes, chez lequel la matière active est incorporée dans la matrice polymère sous la forme d'une prodrogue.

12. Implant selon la revendication 11, chez lequel la matière active est présente fixée dans la matrice polymère par l'intermédiaire de composés chimiques qui sont soumis à une scission par une catalyse basique.

13. Implant selon l'une des revendications précédentes, chez lequel la matière active est choisie dans le groupe contenant des vasodilatateurs, des anti-inflammatoires et des matières actives pour la régulation de la valeur de pH.

14. Implant selon l'une des revendications précédentes, dans lequel la matière active est choisie dans le groupe contenant des substance libérant du NO et le Borsentan®, le dipyridamol, des oligonucléotides synthétiques ODN ou des antagonistes des récepteurs de l'endothéline généraux, des bloqueurs de canaux calciques comme l'amlodipine, la nifidipine ou le verapamil.
